# EUROPEAN PATENT SPECIFICATION

(11) **EP 3 768 385 B1**
(45) Date of publication and mention of the grant of the patent: **29.11.2023**
(21) Application number: 18715528.8
(22) Date of filing: 20.03.2018
(51) Int. Cl.: A61P 17/00, A61K 31/695, A61K 9/06, A61K 9/00, A61P 19/00, A61Q 7/00, A61Q 19/08, A61K 31/663, A61K 47/42, A61K 8/25, A61K 33/06, A61K 36/185, A61K 47/32, A61P 17/14, A61K 31/593, A61Q 3/00

(54) **STABLE, BIOAVAILABLE SILICON COMPLEX FOR MEDICAL USE**
STABILER, BIOVERFÜGBARER SILICIUMKOMPLEX FÜR MEDIZINISCHE VERWENDUNG
COMPLEXE DE SILICIUM BIODISPONIBLE STABLE À USAGE MÉDICAL

(43) Date of publication of application: 27.01.2021
(73) Proprietor: Sil'Innov SCRL, 6180 Courcelles (BE)
(72) Inventor: COSTE-MANIERE, Ivan, 06130 Grasse (FR); CROIZET, Karine, 1490 Court St Etienne (BE)
(74) Representative: Brantsandpatents bv
(86) International application number: PCT/EP2018/057073
(87) International publication number: WO 2019/179611

(56) References cited:
- EP-A1- 2 526 954
- WO-A1-2018/037115
- US-A1- 2017 020 915
- LIDIANE ADVINCULA DE ARAÚJO ET AL: "Use of silicon for skin and hair care: an approach of chemical forms available and efficacy", ANAIS BRASILEIROS DE DERMATOLOGIA, vol. 91, no. 3, 1 June 2016 (2016-06-01), pages 331-335, XP055522162, DOI: 10.1590/abd1806-4841.20163986

## Description

### TECHNICAL FIELD

The present invention relates to the field of silicon compounds for medical use. More specifically, the present invention relates to the field of compounds for the prevention and treatment of injuries, wounds and disorders of keratin and collagen tissue in mammals.

### INTRODUCTION

Pharmaceutical research aims to provide new compounds, compositions and therapies that ensure healing of a medical condition in a human or animal. Importantly, such compounds and compositions need to be administered safely to the subject treated. Many pharmaceutical compounds are known to evoke undesired side effects in the patient's body.

EP 2 526 954 describes a silicon complex formed by reacting orthosilicic acid having four free hydroxy groups and at least one stabilizing agent based on phenol or polyphenol, where the free hydroxy groups of orthosilicic acid are stabilized by hydrogen bonding. The disclosure further relates to a liquid biological preparation comprising said silicon complex, where the liquid biological preparation exhibits a pH of 2-4, the amount of silicon is 0.3-1.4 wt./vol.% and the ratio between the amount of silicon and stabilizing agent is 0.1:1. EP 2 526 954, however, does not describe any beneficial therapeutic and non-therapeutic uses of the claimed compounds and composition.

WO2018/037115 A1 discloses an aqueous composition comprising a silicon complex for use in cosmetics or medicine medicine. The silicon complexes according to this document are structurally different from the claimed ones.

US2017/020915 A1 refers to the use of silicon containing material for treating inflammatory diseases such as alopecia or otitis.

The present invention aims to provide new, safe medical uses of said silicon complex, previously undisclosed to the skilled person.

### SUMMARY OF THE INVENTION

The current invention provides in a solution for at least one of the above mentioned problems by providing a stable, bioavailable silicon complex for medical use.

In a first aspect, the present invention provides a composition for use according to claim 1. In particular, this relates to a composition comprising a stable, bioavailable silicon complex formed between silicic acid having free hydroxyl groups and at least one stabilizing agent based on phenol or polyphenol, for stabilizing at least part of said free hydroxyl groups of silicic acid, for use in the prevention and/or treatment of an injury, a disease, or a disorder in a connective tissue in a subject in need thereof.

In a second aspect, the present invention provides a non-therapeutic process for inducing and/or stimulating growth of human connective tissue, especially skin, hair and nails, comprising the step of administering topically and/or orally to a subject a composition comprising a stable, bioavailable silicon complex formed between silicic acid having free hydroxyl groups and at least one stabilizing agent based on phenol or polyphenol, for stabilizing at least part of said free hydroxyl groups of silicic acid.

### DESCRIPTION OF THE FIGURES

The figures and symbols contained therein have the meaning as commonly understood by one of ordinary skill in the art to which this invention belongs.
Figure 1 shows results of wound healing tests, wherein the length of a wound is depicted as a function of treatment time.
Figure 2 shows results of wound healing tests, wherein the width of a wound is depicted as a function of treatment time.
Figure 3 shows results of wound healing tests, wherein the swelling of tissue around a wound is depicted as a function of treatment time.
Figure 4 shows results of wound healing tests, wherein the visibility of a wound is depicted as a function of treatment time.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight and are abbreviated as "%wt.", unless otherwise defined or unless a different meaning is obvious to the person skilled in the art from its use and in the context wherein it is used.

The terms "additional therapeutically active compound" or "additional therapeutic agent", as used in the context of the present disclosure, refers to the use or administration of a compound for an additional therapeutic use for a particular injury, disease, or disorder being treated. Such a compound, for example, could include one being used to treat an unrelated disease or disorder, or a disease or disorder which may not be responsive to the primary treatment for the injury, disease or disorder being treated. Disease and disorders being treated by the additional therapeutically active agent include, for example, hypertension and diabetes. The additional compounds may also be used to treat symptoms associated with the injury, disease or disorder, including pain and inflammation.

As use herein, the terms "administration of" and or "administering" a compound should be understood to mean providing a compound of the disclosure or a prodrug of a compound of the disclosure to a subject in need of treatment.

The term "antimicrobial agents" as used herein refers to any naturally-occurring, synthetic, or semi-synthetic compound or composition or mixture thereof, which is safe for human or animal use as practiced in the methods described herein, and is effective in killing or substantially inhibiting the growth of microbes. "Antimicrobial" as used herein, includes antibacterial, antifungal, and antiviral agents.

As used herein "burn" or "burns" refer to any detectable injury to tissue caused by energy applied to the tissue. The terms "burn" or "burns" further refer to any burning, or charring of the tissue, including thermal burns caused by contact with flames, hot liquids, hot surfaces, and other sources of high heat as well as steam, chemical burns, radiation, and electrical burns. First degree burns show redness; second degree burns show vesication; third degree burns show necrosis through the entire skin. Burns of the first and second degree are partial-thickness burns, those of the third degree are full-thickness burns.

The term "silicic acid" is to be understood as is the general name for a family of chemical compounds containing the element silicon attached to oxide and hydroxyl groups. This family of compounds has the general formula [SiOₓ(OH)_{4-2*x*}]*ₙ*. Examples include *metasilicic acid* (H₂SiO₃), i.e. the chain or cyclic [SiO(OH)₂]ₙ, *orthosilicic acid* (H₄SiO₄, i.e. Si(OH)₄ with calculated p*K*_{*a*1}=9.84, p*K*_{*a*2}=13.2 at 25 °C), *disilicic acid* (H₂Si₂O₅), i.e. the polymer [SiO_{1.5}(OH)]ₙ, and *pyrosilicic acid* (H₆Si₂O₇), i.e. O(Si(OH)₃)₂. Preferably, said silicic acid is orthosilicic acid. The inventors have found that in the context of the present invention orthosilicic acid provides the best performance in the medical uses.

The term "connective tissue" is to be understood as group of tissues in the body that maintain the form of the body and its organs and provide cohesion and internal support. The connective tissues include several types of fibrous tissue that vary only in their density and cellularity, as well as the more specialized and recognizable variants - bone, ligaments, tendons, cartilage, and adipose (fat) tissue (Encycl. Britannica). The connective tissue is one of the four basic types of animal tissue, along with epithelial tissue, muscle tissue, and nervous tissue. Connective tissue is found in between other tissues everywhere in the body, including the nervous system. They support and protect the body. All connective tissue consists of three main components: fibres (elastic and collagenous fibres), ground substance and cells.

The term "keratinous tissue" is to be understood as a tissue composed of or containing keratin, such as hair, horns, claws, hooves, and the outer layer of human or animal skin. Keratin is one of a family of fibrous structural proteins. It is the key structural material making up hair, horns, claws, hooves, and the outer layer of human or animal skin. Keratin is also the protein that protects epithelial cells from damage or stress. Keratin is extremely insoluble in water and organic solvents. Keratin monomers assemble into bundles to form intermediate filaments, which are tough and form strong unmineralized epidermal appendages found in reptiles, birds, amphibians, and mammals.

A "disease" is a state of health of an animal wherein the animal cannot maintain homeostasis, and wherein if the disease is not ameliorated then the animal's health continues to deteriorate. As used herein, normal aging is included as a disease.

A "disorder" in an animal is a state of health in which the animal is able to maintain homeostasis, but in which the animal's state of health is less favourable than it would be in the absence of the disorder. Left untreated, a disorder does not necessarily cause a further decrease in the animal's state of health.

As used herein, an "effective amount" means an amount sufficient to produce a selected effect, such as alleviating symptoms of a disease or disorder. In the context of administering compounds in the form of a combination, such as multiple compounds, the amount of each compound, when administered in combination with another compound(s), may be different from when that compound is administered alone. Thus, an effective amount of a combination of compounds refers collectively to the combination as a whole, although the actual amounts of each compound may vary. The term "more effective" means that the selected effect is alleviated to a greater extent by one treatment relative to the second treatment to which it is being compared.

The term "improve," as used herein, refers to the ability of a compound, agent, or method to improve a described symptom or condition based on the context in which the term "improve" is used. Preferably, improvement is by at least 10%, more preferably by at least 25%, even more preferably by at least 50%, and most preferably, the symptom or condition is improved by at least 75%.

As used herein, "injury" generally refers to damage, harm, or hurt; usually applied to damage inflicted on the body by an external force.

As used herein, an "instructional material" includes a publication, a recording, a diagram, or any other medium of expression which can be used to communicate the usefulness of a compound of the disclosure in the kit for effecting alleviation of the various diseases or disorders recited herein. Optionally, or alternately, the instructional material may describe one or more methods of alleviating the diseases or disorders in a subject. The instructional material of the kit may, for example, be affixed to a container which contains the identified compound disclosure or be shipped together with a container which contains the identified compound. Alternatively, the instructional material may be shipped separately from the container with the intention that the instructional material and the compound be used cooperatively by the recipient.

"Permeation enhancement" and "permeation enhancers" as used herein relate to the process and added materials which bring about an increase in the permeability of skin to a poorly skin permeating pharmacologically active agent, i.e., so as to increase the rate at which the drug permeates through the skin and enters the bloodstream. "Permeation enhancer" is used interchangeably with "penetration enhancer".

The term "pharmaceutical composition" shall mean a composition comprising at least one active ingredient, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

As used herein, the term "pharmaceutically-acceptable carrier" means a chemical composition with which an appropriate compound or derivative can be combined and which, following the combination, can be used to administer the appropriate compound to a subject.

The term "prevent," as used herein, means to stop something from happening, or taking advance measures against something possible or probable from happening. In the context of medicine, "prevention" generally refers to action taken to decrease the chance of getting a disease or condition.

A "prophylactic" treatment is a treatment administered to a subject who does not exhibit signs of a disease or injury or exhibits only early signs of the disease or injury for the purpose of decreasing the risk of developing pathology associated with the disease or injury.

The term "reduce," as used herein, refers to the ability of a compound, agent, or method to reduce or impede a described symptom based on the context in which the term "reduce" is used. Preferably, reduction is by at least 10%, more preferably by at least 25%, even more preferably by at least 50%, and most preferably, the symptom or condition is reduced by at least 75%.

The term "skin," as used herein, refers to the commonly used definition of skin, e.g., the epidermis and dermis, and the cells, glands, mucosa, and connective tissue which comprise the skin.

A "subject" of diagnosis or treatment is a mammal, including a human.

As used herein, a "subject in need thereof" is a patient, animal, mammal, or human, who will benefit from the treatment of this disclosure.

The term "symptom," as used herein, refers to any morbid phenomenon or departure from the normal in structure, function, or sensation, experienced by the patient and indicative of disease.

A "therapeutic" treatment is a treatment administered to a subject who exhibits signs of pathology for the purpose of diminishing or eliminating those signs.

A "therapeutically effective amount" of a compound is that amount of compound which is sufficient to provide a beneficial effect to the subject to which the compound is administered.

The term "thermal injury" is used interchangeably with "thermal burn" herein.

"Tissue" means (1) a group of similar cells united to perform a specific function; (2) a part of an organism consisting of an aggregate of cells having a similar structure and function; or (3) a grouping of cells that are similarly characterized by their structure and function, such as skin or bone tissue.

The term "topical application," as used herein, refers to administration to a surface, such as the skin. This term is used interchangeably with "cutaneous application" in the case of skin. A "topical application" is a "direct application".

By "transdermal" delivery is meant delivery by passage of a drug through the skin or mucosal tissue and into the bloodstream. Transdermal also refers to the skin as a portal for the administration of drugs or compounds by topical application of the drug or compound thereto. "Transdermal" is used interchangeably with "percutaneous."

As used herein, the term "treating" may include prophylaxis of the specific injury, disease, disorder, or condition, or alleviation of the symptoms associated with a specific injury, disease, disorder, or condition and/or preventing or eliminating said symptoms. A "prophylactic" treatment is a treatment administered to a subject who does not exhibit signs of a disease or exhibits only early signs of the disease for the purpose of decreasing the risk of developing pathology associated with the disease. "Treating" is used interchangeably with "treatment" herein.

As used herein "wound" or "wounds" may refer to any detectable break in the tissues of the body, such as injury to skin or to an injury or damage, or to a damaged site associated with a disease or disorder. Although the terms "wound" and "injury" are not always defined exactly the same way, the use of one term herein, such as "injury", is not meant to exclude the meaning of the other term.

The types of injuries, disease, and disorders encompassed by the methods of the present disclosure therefore include, burns, chronic wounds, and surgical procedures such as microvascular surgery, skin flaps and skin grafts, and tissue injury resulting from, for example, a burn, scrape, cut, incision, laceration, ulcer, body piercing, bite wound, trauma, stab wound, gunshot wound, surgical wound, stretch injury, crush wound, compression wound, fracture, sprain, strain, stroke, infarction, aneurysm, herniation, ischemia, fistula, dislocation, radiation, cell, tissue or organ grafting, injuries sustained during medical procedures, or cancer.

Such injuries include skin injury, muscle injury, brain injury, eye injury, or spinal cord injury. Tissue injury can include joint injury, back injury, heart injury, vascular system injury, soft tissue injury, cartilage injury, lymphatic system injury, tendon injury, ligament injury, or abdominal or gastro-intestinal injuries.

The injuries that are contemplated to be treated by use of a composition of the present invention include, for example, any denuded area without skin or mucosa that is due to trauma such as a burn, a surgical trauma, an abrasion, a malignancy, an infection, or an allergic reaction. It is believed that the use of the composition of present invention will result in an improved cosmetic and functional outcome for the subjects being treated.

The invention can be used to treat all types of thermal injuries and burns. These include acute conditions such as thermal burns, chemical burns, radiation burns, mechanical burns (frictions), burns caused by excess exposure to ultraviolet radiation such as sunburn, as well as by the chronic wounds associated with some of these conditions.

Burns include first degree burns which may cause skin manifestations such as reddening, pain, and/or mild swelling. One non-limiting example of first degree burn is a sun burn. Burns further refers to second-degree burns involving the first two layers of skin. Signs of second degree burning include, among other things, deep reddening of the skin, blisters, pain, glossy appearance from leaking fluid, and possible skin loss. Burns further refers to third-degree burns which penetrate the entire thickness of the skin and may destroy tissue. Signs of third degree burning include, among other things, loss of skin, dry skin, leathery skin, charred skin having a mottled appearance, and combinations thereof. In some cases, skin with a third degree burn may be painless.

It is contemplated that the compositions of the present invention will benefit, for example, subjects suffering from vesicant burns and thermal burns, including first degree burns, second degree burns and third degree burns, as well as esophageal and gastro-intestinal burns and erosions. For example, after cutaneous burn injury, an area surrounding the wound is the site of a pronounced inflammatory response with an associated reduced blood flow. This "zone of stasis" undergoes progressive necrosis within 24-48 hours resulting in an expansion of the burn wound characterized by decreased microvascular blood flow.

Injuries encompassed herein further include acute and chronic wounds. Chronic wounds are wounds characterized by non-healing skin wounds and include chronic venous ulcers, diabetic ulcers, arterial ulcers, pressure ulcers (e.g., decubitus ulcers), radiation ulcers, traumatic wounds, and open, complicated non-healing wounds. Wounds further refers to cuts and scrapes known as open wounds, as well as others, such as deep bruises, or closed wounds. Non-limiting examples of wounds suitable for treatment in accordance with the present disclosure include abrasions such as those caused by: scraping the outer layer of skin; incisions such as those caused by sharp edges, knives, metal edges, broken glass or other sharp object; lacerations or jagged, irregular cuts or tears of the skin; punctures such as those caused by an object piercing the skin layers and creating a small hole; and/or burns. Additional non-limiting wounds suitable for treatment in accordance with the present disclosure include puncture wounds, gaping wounds, wounds having fatty layers, tissue or muscle exposed, wounds having one or more foreign bodies therein, wounds causing severe pain, wounds having blood flowing there from, or any wound that causes numbness or loss of movement below the wound.

Other non-limiting examples of wounds suitable for treatment in accordance with the present disclosure include animal bites, arterial disease, insect stings and bites, bone infections, compromised skin/muscle grafts, gangrene, skin tears or lacerations, surgical incisions, including slow or non-healing surgical wounds, and post-operation infections. It is understood, that the listed wounds are non-limiting and that only a portion of wounds suitable for treatment in accordance with the present disclosure are listed herein.

It is also contemplated that the composition of the present invention will benefit subjects with chronic skin ulcers, including but limited to decubitus ulcers, venous stasis ulcers, arterial insufficiency ulcers, and diabetic foot ulcers.

As used herein, "additional ingredients" include one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions are known in the art and described, for example in Genaro, ed. (1985, Remington's Pharmaceutical Sciences, Mack Publishing Co., Easton, Pa.).

Other components such as preservatives, antioxidants, surfactants, absorption enhancers, viscosity enhancers or film forming polymers, bulking agents, diluents, colouring agents, flavouring agents, pH modifiers, sweeteners or taste-masking agents may also be incorporated into the composition. Suitable colouring agents include, but are not limited to, red, black, and yellow iron oxides and FD&C dyes such as FD&C Blue No. 2 and FD&C Red No. 40. Suitable flavouring agents include mint, raspberry, licorice, orange, lemon, grapefruit, caramel, vanilla, cherry grape flavours and combinations thereof. Suitable pH modifiers include, but are not limited to, citric acid, tartaric acid, phosphoric acid, hydrochloric acid, maleic acid and sodium hydroxide. Suitable sweeteners include, but are not limited to, aspartame, acesulfame K and thaumatin. Suitable taste-masking agents include, but are not limited to, sodium bicarbonate, vanilla, ion-exchange resins, cyclodextrin inclusion compounds and adsorbates.

Absorption enhancers for use in accordance with the present disclosure include, for example, polysorbates, sorbitan esters, poloxamer block copolymers, PEG-35 castor oil, PEG-40 hydrogenated castor oil, caprylocaproyl macrogol-8 glycerides, PEG-8 caprylic/capric glycerides, sodium lauryl sulfate, dioctyl sulfosuccinate, polyethylene lauryl ether, ethoxydiglycol, propylene glycol mono-dicaprylate, glycerol monocaprylate, glyceryl fatty acids, oleic acid, linoleic acid, glyceryl caprylate/caprate, glyceryl monooleate, glyceryl monolaurate, caprylic/capric triglycerides, ethoxylated nonylphenols, PEG-(8-50) stearates, olive oil PEG-6 esters, triolein PEG-6 esters, lecithin, d-alpha tocopheryl polyethylene glycol 1000 succinate, polycarbonate, sodium glycocholate, sodium taurocholate, cyclodextrins, citric acid, sodium citrate, triacetin and combinations thereof. A preferred absorption enhancer is triacetin. A preferred absorption enhancer wherein an absorption enhancer is included in the formulation, the absorption enhancer is included in an amount of from about 0.001% to about 10% by weight of the formulation, preferably in an amount of about 0.01% to about 5% by weight of the formulation.

### Medical uses

In a first aspect, the present invention provides a composition comprising a stable, bioavailable silicon complex formed between silicic acid having free hydroxyl groups and at least one stabilizing agent based on phenol or polyphenol, stabilizing at least part of said free hydroxyl groups of silicic acid, for use in the prevention and/or treatment of an injury, a disease, or a disorder in a connective and/or keratinous tissue in a subject in need thereof. Without being bound to any mechanistic studies, it is assumed that said silicic acid is stabilized by hydrogen bonding and optionally further interactions. Said composition may further comprise one or more pharmaceutically acceptable excipients and/or emollients.

In a preferred embodiment, the present invention provides a composition according to the first aspect of the invention, wherein said silicic acid is orthosilicic acid having four free hydroxyl groups. The inventors have found that orthosilicic acid provides improved results in terms of medical use. In this complex, the orthosilicic acid carries four free hydroxyl groups, which excludes any presence of organic radical and therefore of organosilanes.

The orthosilicic acid is complexed with at least one phenolic or polyphenolic compound comprising at least one aromatic ring and one or more hydroxyl groups. Preferably it could further contain one or more carbonyl groups (C=O). The aromatic, and therefore sterically hindered, structure of phenolic or polyphenolic compounds and the presence of hydroxyl and optionally carbonyl groups play a fundamental role in the stabilization process. Hydrogen bonds characteristic of weak electrostatic bonds are established between the hydroxyl groups of orthosilicic acid and the hydroxyl and carbonyl groups of the phenolic compounds. Although not being bound by any mechanistic theories, it is assumed that an esterification reaction between the orthosilicic acid and the phenolic compound (polyphenolic) is excluded. These bonds prevent the polymerization of orthosilicic acid and the formation of Si-O-Si bonds.

In a preferred embodiment, the present invention provides a composition according to the first aspect of the invention for use in the prevention and/or treatment of sores, wounds, ulcers, fistula or otitis in a subject in need thereof. The composition according to the first aspect of the invention may advantageously be used for prevention and/or treatment of an injury, a disease, or a disorder wherein the said injury, disease, or disorder is selected from the group consisting of thermal injury such as skin burn injury, skin injury, soft tissue injury, non-healing skin wound, burns, acute wound, chronic wound, scrape, cut, incision, laceration, decubitis, pressure ulcer, chronic venous ulcer, venous stasis ulcer, diabetic ulcer, arterial ulcer, radiation ulcer, traumatic wound, open complicated non-healing wound, body piercing, bite wound, insect bite, insect sting, stab wound, gunshot wound, stretch injury, crush wound, compression wound, fracture, sprain, strain, stroke, infarction, aneurism, herniation, ischemia, fistula, dislocation, radiation, or surgery. In a more preferred embodiment, the present invention provides a composition according to the first aspect of the invention wherein said skin burn injury is selected from the group of burns consisting of thermal, radiation, chemical, electrical, steam, and sunburn.

In a preferred embodiment, the present invention provides a composition according to the first aspect of the invention for use for the prevention of joint and bone health loss and/or improvement of joint and bone health.

More specifically, the present invention provides a composition according to the first aspect of the invention for use in the prevention or treatment of joint and bone health loss and/or improvement of joint and bone health, in relation to joint or bone injuries, age-related wear, to autoimmune conditions, for treatment of joint pain, joint redness, stiffness, and swelling, joint dislocation, bunions, carpal tunnel syndrome, Sjogren's syndrome, joint surgery, lupus, menopause and/or hormonal induced joint pain. Also, the present invention provides a composition according to the first aspect of the invention for use in the prevention or treatment of inflammation of joints such as arthritis, osteoporosis, osteoarthritis, psoriatic arthritis, polyarthritis, rheumatoid arthritis, knee bursitis, and vasculitis.

Furthermore, the present invention provides a composition according to the first aspect of the invention for use in the prevention or treatment of collagen density loss and/or improvement of collagen density. More preferably, said collagen constitutes bone collagen and/or skin collagen.

In one embodiment, the present invention provides a composition according to the first aspect of the invention for use for the improvement of joint and bone health in a subject subjected to ovariectomy. More specifically, the present invention provides a composition according to the first aspect of the invention for use in the prevention or treatment of collagen density loss and/or improvement of collagen density wherein said composition is used in combination with supplement of calcium and/or vitamin D. Preferably, said composition is used further in combination with alendronic acid, and a supplement of calcium and/or vitamin D.

In a preferred embodiment, the present invention provides a composition according to the first aspect of the invention for use in the prevention and/or treatment of nail diseases. Preferably, the present invention provides a composition according to the first aspect of the invention for use in the prevention and/or treatment of nail diseases, wherein said nail disease is selected from the group comprising onychia, onychocryptosis, onychodystrophy i.e. resulting from cancer chemotherapy, onychogryposis, onycholysis i.e. caused by internal disorder, trauma, infection, nail fungi, allergy to nail enhancement products, or side effects of drugs, onychomadesis i.e. caused by localized infection, minor injury to the matrix bed, or severe systemic illness or as a side effect of chemotherapy or x-ray treatments for cancer, onychomycosis also known as tinea unguium i.e. caused by fungal infections, onychoptosis i.e. caused by certain diseases such as syphilis, or by fever, trauma, systemic upsets or adverse reaction to drugs, onychorrhexis, paronychia i.e. caused by bacterial or fungal infection, koilonychia i.e. caused by iron deficiency, onychomatricoma, nail pemphigus, erythronychia i.e. caused by inflammatory conditions and melanonychia.

In a preferred embodiment, the present invention provides a composition according to the first aspect of the invention for use in the prevention and/or treatment of alopecia, selected from the group comprising alopecia areata, androgenetic alopecia, postmenopausal alopecia, female pattern alopecia, seborrheic alopecia, alopecia pityroides, senile alopecia, drug-induced alopecia, cancer chemotherapy drug-induced alopecia, alopecia due to radiation exposure, trichotillomania, postpartum alopecia.

### Preferred dosage

In a preferred embodiment, the present invention provides a composition according to the first aspect of the invention whereby said composition is orally administered to a subject in need thereof. Preferably, said composition is administered 1 to 5 times daily, preferably 1 to 3 times daily, in an amount of 0.10 mL per kg body weight to 0.80 mL per kg body weight, preferably in an amount of 0.10 mL per kg body weight to 0.50 mL per kg body weight, and more preferably in an amount between 0.15 mL and 0.40 mL kg body weight and even more preferably between 0.20 mL and 0.30 mL kg body weight. Preferably, said composition is administered before food intake, such as i.e. before breakfast.

In a preferred embodiment, the present invention provides a composition according to the first aspect of the invention wherein said composition is topically, directly administered to a subject in need thereof. Preferably, said composition is administered 1 to 3 times daily, and more preferably twice daily. Preferably, said composition is left in contact with the treated subject for an extended period of time, thereby allowing for proper penetration of the composition within the treated connective and/or keratinous tissue.

The precise dosage administered will vary depending upon any number of factors including the type of animal and type of disease state being treated, the age of the animal and the route of administration. The composition may be administered to a subject as frequently as several times daily, or it may be administered less frequently, such as once a day or once a week. The frequency of the dose will be readily apparent to the skilled artisan and will depend upon any number of factors, such as the type and severity of the disease being treated, the type and age of the animal.

### Preferred composition

The stabilizer is preferably a phenolic or polyphenolic compound and the following compounds, not limited thereto, may be exemplified: phenolic acids, such as 3-4-5-trihydroxybenzoic acid, 4-hydroxy-3- acid; methoxybenzoic acid, 3-4-dihydroxybenzoic acid, 4-hydroxy-3,5-dimethoxybenzoic acid, 2-hydroxybenzoic acid, 2-5-dihydroxybenzoic acid, 3-(4-hydroxyphenyl) acid 2-propenoic acid, 3-(3,4-dihydroxyphenyl) prop-2-enoic acid, 3-(4-hydroxy-3-methoxyphenyl) prop-2-enoic acid, 3-(4-hydroxy-3,5-dimethoxyphenyl)-prop-2-enoic acid, (R)-a-[[3-(3,4-dihydroxyphenyl)-1-oxo-2E-propenyl]-oxy]-3,4-dihydroxybenzenepropanoic acid, etc.; simple phenols, such as benzene-1,2-diol, benzene-1,3-diol, 2-isopropyl-5-methylphenol; phenylbutanone, such as 1-(4-hydroxyphenyl) -3-butanone; aldehyde derivatives of phenolic acids, such as 4-hydroxy-3-methoxybenzaldehyde, etc.; cinnamic aldehydes, such as 4-allyl-2-methoxyphenol, 2-methoxy-4-propenylphenol, etc.; coumarins, such as 7-hydroxychromen-2-one, 6,7-dihydroxychromen-2-one, etc.; naphthoquinones, such as 5-hydroxy-1,4-naphthoquinone, etc.; flavonoids, such as catechin (2-(3,4-dihydroxyphenyl) chroman-3,5,7-triol) and epicatechin (EC), epigallocatechin (EGC), epicatechin gallate (ECG), epigallocatechin gallate (EGCG), kaempferol, quercetol, luteolin, etc.; stilbenes, such as resveratrol, pinosylvin, piceatannol, pterostilbene, etc. Preferred stabilizers are 4-hydroxy-3-methoxybenzaldehyde, 1-(4-hydroxyphenyl)-3-butanone, 2-hydroxybenzoic acid, and (2-(3,4-dihydroxyphenyl)-chromane-3,5,7-triol). In a more preferred embodiment, the present invention provides a composition according to the first aspect of the invention, wherein said stabilizing agent is 4-hydroxy-3-methoxybenzaldehyde.

In a preferred embodiment, the present invention provides a composition according to the first aspect of the invention having a pH of less than 6, less than 5, and preferably less than 4 and greater than or equal to 2, and a Si content of between 0.3 ‰ and 1.4 ‰ by weight per volume of the composition and a ratio between the content of Si (wt./vol.-% of said composition) and the content of stabilizing agent (wt./vol.-% of said composition) of between 0.1 and 1. Preferably, the present invention provides a composition according to the first aspect of the invention having a Si content between 0.4 ‰ and 1.2 ‰ by weight per volume of preparation, more preferably between 0.5 ‰ and 1.1 ‰ by weight per volume of preparation, even more preferably between 0.6 ‰ and 1.0 ‰ by weight per volume of preparation and most preferably between 0.7 ‰ and 0.9 ‰ by weight per volume of preparation. The Si content of the preparation and the ratio% of Si (wt./vol.)-% of stabilizing agent (wt./vol.) indicated above must be respected for the preparation to contain enough bioavailable silicon complex and this without risk of polycondensation. More preferably, the ratio of Si (wt./vol.)-% of stabilizing agent (wt./vol.) is between 0.5 and 0.9, in particular between 0.6 and 0.8.

In a more preferred embodiment, the present invention provides a composition according to the first aspect of the invention, wherein said composition is a stable aqueous-alcoholic solution of said silicon complex having a pH of between 2.5 and 3.5.

The process for preparing such compositions is detailed in EP 2 526 954. Such compositions are also free of organic silicon molecules and halogenated compounds, as well as covalent bonds between the orthosilicic acid and the stabilizing agent. The preparation thus obtained can advantageously have an average silicon content of 0.749 ‰ (wt./vol.), which is equivalent to an average concentration of 26.7 mM Si. According to an advantageous embodiment, the silicon content is 0.859 %o (wt./vol.), i.e., its average concentration is 30.6 mM Si, and the phenolic compound content 0.105% (wt./vol.). The silicon concentration is preferably maintained below 1.1 %o (wt./vol.) so that the polycondensation process is not favoured. Below a concentration of 0.3 ‰ the concentration of Si is no longer sufficient to ensure efficient preparation.

### Preferred excipients and further ingredients

In a preferred embodiment, the present invention provides a composition according to the first aspect of the invention further comprising one or more therapeutic agents. Preferably, said additional therapeutic agent is selected from the group consisting of vitamins, antimicrobial agents, disinfectants, fungicides, anti-inflammatories, wound care products, wound healing agents, plant extracts, animal extracts, cell extracts, antibacterial agents, antifungal agents, antiviral agents, antibiotics. Several preferred embodiments include use of any therapeutic molecule including any pharmaceutical or drug. Examples of pharmaceuticals include sedatives and sleep inducers, antiallergics, antiarthritics, appetite suppressants, muscle relaxants, vitamins, antimicrobial agents, antacids, antiseptics, diuretics, disinfectants, fungicides, ectoparasiticides, antiparasitics, antioxidants, vitamins, cosmetics, anti-inflammatories, wound care products, wound healing agents, plant extracts, emollients, antibacterial agents, antifungal agents, antiviral agents, antibiotics.

A list of the types of drugs, and specific drugs within categories which are encompassed within the invention is provided below and are intended be non-limiting examples. Antimicrobial agents include: silver sulfadiazine, Nystatin, Nystatin/triamcinolone, Bacitracin, nitrofurazone, nitrofurantoin, a polymyxin (e.g., Colistin, Surfactin, Polymyxin E, and Polymyxin B), doxycycline, antimicrobial peptides (e.g., natural and synthetic origin), Neosporin (i.e., Bacitracin, Polymyxin B, and Neomycin), Polysporin (i.e., Bacitracin and Polymyxin B). Additional antimicrobials include topical antimicrobials (i.e., antiseptics), examples of which include silver salts, iodine, benzalkonium chloride, alcohol, hydrogen peroxide, and chlorhexidine. Anti-inflammatory: Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium.

In a preferred embodiment, the present invention provides a composition according to the first aspect of the invention further comprising a skin permeation agent and/or a skin permeation enhancer, especially for a topical application dosage form. These materials increase the rate of penetration of the composition according to the invention across the skin. Typical enhancers in the art include ethanol, glycerol monolaurate, PGML (polyethylene glycol monolaurate) and dimethylsulfoxide. Other enhancers include oleic acid, oleyl alcohol, ethoxydiglycol, laurocapram, alkanecarboxylic acids, dimethylsulfoxide, polar lipids, or N-methyl-2-pyrrolidone.

A composition for topical use may further comprise other ingredients such as moisturizers, cosmetic adjuvants, antioxidants, chelating agents, bleaching agents, tyrosinase inhibitors, and other known depigmentation agents, surfactants, foaming agents, conditioners, humectants, wetting agents, emulsifying agents, fragrances, viscosifiers, buffering agents, preservatives and sunscreens. In another example, a permeation or penetration enhancer is included in the composition and is effective in improving the percutaneous penetration of the active ingredient into and through the stratum corneum with respect to a composition lacking the permeation enhancer. Various permeation enhancers, including oleic acid, oleyl alcohol, ethoxydiglycol, laurocapram, alkanecarboxylic acids, dimethylsulfoxide, polar lipids, or N-methyl-2-pyrrolidone, are known to those of skill in the art. The composition may further comprise a hydrotropic agent, which functions to increase disorder in the structure of the stratum corneum, and thus allows increased transport across the stratum corneum. Various hydrotropic agents such as isopropyl alcohol, propylene glycol, or sodium xylene sulfonate, are known to those of skill in the art.

Modification of compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include humans and other primates, mammals including commercially relevant mammals such as cattle, pigs, horses, sheep, cats, and dogs.

### Preferred dosage form

In a preferred embodiment, the present invention provides a composition according to the first aspect of the invention, wherein said composition is a formulation selected from the group consisting of liquid or semi-liquid preparations such as liniments, lotions, oil-in-water or water-in-oil emulsions such as creams, ointments or pastes, and solutions or suspensions.

The formulations described herein may be prepared in a variety of forms known in the art, such as liquids, aerosols, or gels. Topical administration of the present formulation can be performed by, for example, hand, mechanically (e.g., extrusion and spray delivery) or as a component of a dressing (e.g., gauze or other wound covering). The administration of the formulation directly by hand to a tissue or biomaterial surface is performed so as to achieve a therapeutic coating, which may be uniform, alone or in combination with an overlying dressing.

Formulations suitable for topical administration include liquid or semi-liquid preparations such as liniments, lotions, oil-in-water or water-in-oil emulsions such as creams, ointments or pastes, and solutions or suspensions. Formulations for topical administration may further comprise one or more of the additional ingredients described herein.

An obstacle for topical administration of pharmaceuticals to the skin is the stratum corneum layer of the epidermis. The stratum corneum is a highly resistant layer comprised of protein, cholesterol, sphingolipids, free fatty acids and various other lipids, and includes cornified and living cells. One of the factors that limits the penetration rate (flux) of a compound through the stratum corneum is the amount of the active substance which can be loaded or applied onto the skin surface. The greater the amount of active substance which is applied per unit of area of the skin, the greater the concentration gradient between the skin surface and the lower layers of the skin, and in turn the greater the diffusion force of the active substance through the skin. Therefore, a formulation containing a greater concentration of the active substance is more likely to result in penetration of the active substance through the skin, and more of it, and at a more consistent rate, than a formulation having a lesser concentration, all other things being equal.

Additionally, formulations for topical administration may include liquids, ointments, lotions, creams, gels (e.g., poloxamer gel), sprays and aerosols. Conventional pharmaceutical carriers, aqueous bases and thickeners may be necessary or desirable. The disclosed compositions can be administered, for example, in a microfiber, polymer (e.g., collagen), aerosol, lotion, cream, fabric, tissue engineered scaffold, resin, wound dressing, capsule, injectables, intravenous drips, silicone implants, or any bio-engineered materials.

Suitable solvents for the composition according to the first aspect of the invention can be water, aliphatic and aromatic alcohols, sulfoxides, fatty acids, fatty acid esters, polyols, amides, surfactants, terpenes, alkanones, organic acids and mixtures thereof. Suitable alcohols include, without limitation, ethanol, propanol, butanol, pentanol, hexanol, octanol, nonanol, decanol, 2-butanol, 2-pentanol, benzyl alcohol, phenoxyethanol, caprylic alcohol, decyl alcohol, lauryl alcohol, 2-lauryl alcohol, myristyl alcohol, cetyl alcohol, stearyl alcohol, oleyl alcohol, linolyl alcohol, linolenyl alcohol and mixtures thereof. Volatile aliphatic alcohols having 2 to about 8 carbon atoms, preferably 2 to about 5 carbon atoms, can provide a dual function of serving both as volatile carrier and penetration enhancer. The aromatic alcohols, such as benzyl alcohol, phenoxyethanol, and the like can provide a dual function of serving both as a substantially non-volatile, permeation enhancer and auxiliary anti-infective. Preferred alcohols are ethanol and benzyl alcohol. Suitable sulfoxides include dimethylsulfoxide, decylmethylsulfoxide, and mixtures thereof. Suitable fatty acids include valeric, heptanoic, pelargonic, caproic, capric, lauric, myristic, stearic, oleic, linoleic, linolenic, caprylic, isovaleric, neopentanoic, neoheptanoic, neononanoic, trimethyl hexanoic, neodecanoic and isostearic acids, and mixtures thereof. Suitable fatty acid esters include isopropyl n-butyrate, isopropyl n-hexanoate, isopropyl n-decanoate, isopropyl myristate, isopropyl palmitate, octyidodecyl myristate, ethyl acetate, butyl acetate, methyl acetate, methylvalerate, methylpropionate, diethyl sebacate, ethyl oleate, ethyl laurate and mixtures thereof. Suitable polyols include propylene glycol, polyethylene glycol, ethylene glycol, diethylene glycol, triethylene glycol, dipropylene glycol, glycerol, propanediol, sorbitol, dextrans, butanediol, pentanediol, hexanetriol, and mixtures thereof. Suitable amides include urea, dimethylacetamide, diethyltoluamide, dimethylformamide, dimethyloctamide, dimethyldecamide, pyrrolidone derivatives, 1-alkyl-4-imidazolin-2-one, cyclic amides, hexamethylenelauramide and its derivatives, diethanolamine, triethanolamine and mixtures thereof. Suitable pyrrolidone derivatives includel-methyl-2-pyrrolidone, 2-pyrrolidone, 1-lauryl-2-pyrrolidone, 1-lauryl-4-carboxy-2-pyrrolidone, 1-methyl-4-carboxy-2-pyrrolidone, 1-hexyl-4-carboxy-2-pyrrolidone, 1-decylthioethyl-2-pyrrolidone, N-cyclohexyl-pyrrolidone, 1-methyl-4-methoxycarbonyl-2-pyrrolidone, 1-hexyl-4-methoxycarbonyl-2-pyrrolidone, 1-lauryl-4-methoxycarbonyl-2-pyrrolidone, N-dimethylamino-propyl-pyrrolidone, N-cocoylpyrrolidone, N-tallowylpyrrolidone, fatty acid esters of N-(2-hydroxymethyl)-2-pyrrolidone, and mixtures thereof. Suitable cyclic amides include, 1-dodecylazacycloheptan-2-one, 1-geranylazacycloheptan-2-one, 1-farnesylazacycloheptan-2-one, 1-geranyl-geranyl-azacycloheptan-2-one, 1-(3,7-dimethyloctyl) azacycloheptan-2-one, 1-(3,7,11-trimethyl-octyl) azacycloheptan-2-one, 1-geranylazacyclohexan-2-one, 1-geranyl-azacyclopentan-2,5-dione, 1-farnesylazacyclopentan-2-one, and mixtures thereof. Suitable surfactants include anionic surfactants, cationic surfactants, nonionic surfactants, amphoteric surfactants and lecithin. Suitable anionic surfactants include sodium laurate, sodium lauryl sulfate, and mixtures thereof. Suitable cationic surfactants include cetyltrimethylammonium bromide, tetradecyltrimethyl ammonium bromide, benzalkonium chloride, octadecyltrimethyl ammonium chloride, cetylpyridinium chloride, dodecyltrimethylammonium chloride, hexadecyltrimethylammonium chloride, and mixtures thereof. Suitable nonionic surfactants include alpha-hydro-(D-hydroxy poly(oxyethylene)-poly(oxypropyl) poly(oxyethylene) block copolymers, polyoxyethylene ethers, polyoxyethylene sorbitan esters, polyethylene glycol esters of fatty alcohols, and mixtures thereof. Suitable alpha-hydro-co-hydroxy-poly(oxyethylene)-poly(oxypropyl) poly (oxyethylene) block copolymers include Poloxamers 182, 184, 231, and mixtures thereof. Suitable polyethylene glycol esters of fatty acids include polyoxyethylene, polyoxyethylene monostearate, the polyoxyethylene monostearate and mixtures thereof. Suitable amphoteric surfactants include, without limitation thereto, lauramidopropyl betaine, cocamidopropyl betaine, lauryl betaine, cocobetaine, cocamidopropyl-hydroxy-sultaine, aminopropyl laurylglutamide, sodium cocoamphoacetate, sodium lauro-amphoacetate, disodium lauroamphodiacetate, disodium cocoamphodiacetate, sodium-cocoamphopropionate, disodium lauroamphodipropionate, disodium cocoampho-dipropionate, sodium-lauriminodipropionate, disodium-cocoampho-carboxy-methylhydroxy-propylsulfate, and the like. Suitable terpenes include D-limonene, α-pinene, β-enrene, α-terpineol, terpinen-4-ol, carvol, carvone, pulegone, piperitone, menthon, menthol, geraniol, cyclohexene oxide, limonene oxide, α-pinne oxide, cyclopentene oxide, 1,8-cineol, ylang ylang oil, anise oil, chenopodium oil, eucalyptus oil, and mixtures thereof. Suitable alkanones include N-heptane, N-octane, N-nonane, N-decane, N-undecane, N-dodecane, N-tridecane, N-tetradecane, N-hexadecane, and mixtures thereof. Suitable organic acids include citric acid, succinic acid, salicylic acid, salicylates (including the methyl, ethyl and propyl glycol derivatives), tartaric acid, and mixtures thereof.

Topical administration of compositions of the invention may include transdermal application. Transdermal application can be performed either passively or using iontophoresis or electroporation.

Compositions of the invention may be applied using transdermal patches. Transdermal patches are adhesive backed patches laced with an effective amount of compounds of the invention. The pressure-sensitive adhesive of the matrix will normally be a solution of polyacrylate, a silicone, or polyisobutylene (PIB). Such adhesives are well known in the transdermal art. See, for instance, the Handbook of Pressure Sensitive Adhesive Technology, 2nd Edition (1989) Van Nostrand, Reinhold.

Dosage forms for topical or transdermal administration of a compound of this disclosure include liquids, ointments, pastes, creams, lotions, gels, solutions, sprays, aerosols or patches. The active component is admixed under sterile conditions with a pharmaceutically acceptable carrier and any needed preservatives or buffers as may be required. Transdermal patches have the added advantage of providing controlled delivery of a compound to the body. Such dosage forms can be made by dissolving or dispensing the compound(s) in the proper medium. Absorption enhancers can also be used to increase the flux of the compound across the skin. The rate can be controlled by either providing a rate controlling membrane or by dispersing the compound in a polymer matrix or gel.

The ointments, pastes, creams, and gels may contain, in addition to an active compound of this disclosure excipients such as animal and vegetable fats, oils, waxes, paraffins, starch, tragacanth, cellulose derivatives, polyethylene glycols, silicones, bentonites, silicic acid, talc and zinc oxide, or mixtures thereof.

### Method for use of prevention and/or treatment

In a further aspect, the present invention provides a method for use in preventing and/or
treating an injury, a disease, or a disorder in a connective tissue in a subject in need thereof - as described above -, said method comprising the step of administering to a subject in need thereof a composition according to the first aspect of the invention.

In a preferred embodiment, the present invention provides said method wherein said composition is orally administered to a subject in need thereof. Preferably, said composition is administered 1 to 5 times daily in an amount of 0.10 mL per kg body weight to 0.50 mL per kg body weight, and more preferably in an amount between 0.15 mL and 0.40 mL kg body weight and even more preferably between 0.20 mL and 0.30 mL kg body weight. Preferably, said composition is administered before food intake, such as i.e. before breakfast.

In an alternative or complementary embodiment, the present invention provides said method wherein said composition is topically administered to a subject in need thereof. Preferably, said composition is administered 1 to 3 times daily, and more preferably twice daily. Preferably, said composition is left in contact with the treated subject for an extended period of time, thereby allowing for proper penetration of the composition within the treated connective and/or keratinous tissue.

The precise dosage administered will vary depending upon any number of factors including the type of animal and type of disease state being treated, the age of the animal and the route of administration. The composition may be administered to a subject as frequently as several times daily, or it may be administered less frequently, such as once a day or once a week. The frequency of the dose will be readily apparent to the skilled artisan and will depend upon any number of factors, such as the type and severity of the disease being treated, the type and age of the animal.

### Non-therapeutic use

In a second aspect, the present invention provides a non-therapeutic, cosmetic method for improving connective and/or keratinous tissue, especially skin, hair and nails, comprising the step of administering topically and/or orally to a subject, more specifically to a mammal, a composition comprising a stable, bioavailable silicon complex formed between silicic acid having free hydroxyl groups, preferably orthosilicic acid having four free hydroxyl groups, and at least one stabilizing agent based on phenol or polyphenol, stabilizing said free hydroxyl groups of said silicic acid. Such a method has been shown to visibly improve the appearance of connective and/or keratinous tissue and induces and/or stimulates the growth of connective and/or keratinous tissue. Preferably, said method comprises the step of leaving said composition in contact with connective and/or keratinous tissue for a period of time and optionally rinsing said composition from said connective and/or keratinous tissue. Such methods have proven to improve nail growth, halt or reduce loss of hair and/or increasing density of hair, as well as reducing wrinkle visibility.

In a preferred embodiment, the present invention provides a non-therapeutic process according to the second aspect of the invention, whereby said composition is orally administered to a subject in need thereof. Preferably, said composition is administered 1 to 3 times daily in an amount of 0.10 mL per kg body weight to 0.50 mL per kg body weight, and more preferably in an amount between 0.15 mL and 0.40 mL kg body weight and even more preferably between 0.20 mL and 0.30 mL kg body weight. Preferably, said composition is administered before food intake, such as i.e. before breakfast.

In a preferred embodiment, the present invention provides a non-therapeutic process according to the second aspect of the invention, whereby said composition is topically administered to a subject in need thereof. Preferably, said composition is administered 1 to 3 times daily, and more preferably twice daily. Preferably, said composition is left in contact with the treated subject for an extended period of time, thereby allowing for proper penetration of the composition within the treated connective and/or keratinous tissue.

The precise dosage administered will vary depending upon any number of factors including the age of the subject and the route of administration. The composition may be administered to a subject as frequently as several times daily, or it may be administered less frequently, such as once a day or once a week. The frequency of the dose will be readily apparent to the skilled artisan in the context of the present disclosure.

### EXAMPLES

In the following examples are intended to further clarify the present invention, and are nowhere intended to limit the scope of the present invention.

### EXAMPLE 1

Extra pure vanillin with purity guaranteed to 99.9% is used. The alternatives "Fine Mesh" or "Free flow" will be preferred. 76 g of vanillin powder are dissolved with stirring at 40°C in 100 ml of 40% ethanol. The homogeneous mixture obtained is diluted slowly with osmosis demineralized water so that the final volume ratio of the solution per volume of sodium orthosilicate introduced is equal to 225. The pH is then adjusted between 1.5 and 2.5 with a solution of phosphoric acid 14.5 N. Sodium orthosilicate (428 g, density: 1.35) is then added to the mixture, dropwise, with stirring. The solution is kept under constant stirring. Upon addition of the sodium orthosilicate, the temperature is maintained in a range of 10 to 15°C. The final pH is adjusted between 2.5 and 3.5 with 30% (w/w) phosphoric acid solution.

The resulting solution is clear and colourless. It has a Si content of 0.86 %o and a % Si (w/v)/% vanillin (w/v) ratio of 0.8.

### EXAMPLE 2

A 100 mL orally administrable organic silicium solution according to the invention comprises purified water, silicic acid (233 mg including 66.7 mg of silicon), hydro alcoholic nettle/urtica dioica extract (7.5 mg), methane methyl sulphonate (3330 mg), D-glucosamine sulphate (3333 mg), chondroitin sulphate (1774 mg), citrus sinensis/sweet orange essential oil (50 mg), xylitol, sucralose, flavourings (vanillin, grapefruit), gum Arabic, copper gluconate, zinc gluconate, phosphoric acid.

### EXAMPLE 3

A topically applicable organic silicium gel according to the invention comprises water (74.148%), monomethylsilanetriol (0.064%), methylsulphonylmethane (5%), isopropyl alcohol (5%), flavouring (3.1%), capryloyl glycine (2%), simmondsia chinensis seed cera (1.5%), tromethamine (1.2%), benzyl alcohol (1%), polyacrylate crosspolymer-6 (1%), hydroxyethyl acrylate/sodium acryloyldimethyl taurate copolymer (1%), glucosamine HCl (0.212%), sodium chondroitin sulphate (0.101%), citrus sinensis peel extract (0.6%), mentha piperita herb oil (0.02%), polyacrylate 13 (0.68%), acacia gum (0.6%), xanthan gum (0.6%), xylitylglucoside (0.5%), anhydroxylitol (0.3%), polyisobutene (0.27%), xylitol (0.2%), hydrolyzed milk protein (0.143%), polysorbate-20 (0.05%).

### EXAMPLE 4

A 100 ml orally administrable silicium solution is prepared in accordance to the instructions detailed in Example 1 and comprises purified water, silicic acid (292 mg including 86 mg of silicon), hydroalcoholic purified nettle/urtica dioïca extract (7.5 mg), flavourings (vanillin, lemon), phosphoric acid.

Female mice subjected to ovariectomy were studied with regard to bone loss using microscanner analysis, and were compared to a control group of sham operated female mice (Group I, 8 subjects). After performing the ovariectomy, one group of mice was treated using a control supplement comprising a vehicle solution (Group II, 8 subjects); one group of mice was treated with a daily dose of 1.32 mg Si per kg body weight of the siliceous solution described above (Group III, 10 subjects); one group of mice was treated with a daily dose of calcium and vitamin D3 supplement (Group IV, 10 subjects); one group of mice was treated with a daily dose of 1.32 mg Si per kg body weight of the siliceous solution described above and a daily dose of calcium and vitamin D3 supplement (Group V, 10 subjects). Results of the bone loss study are compiled in Table 1 below.

**Table 1. Bone loss in control group and in female mice subjected to ovariectomy.**

| | BMD ^{a} | BV/VT ^{b} | TbTh ^{c} | TbN ^{d} |
|---|---|---|---|---|
| Group I | 431 | 38.34 | 118 | 3.22 |
| Group II | 295 | 19.27 | 103 | 1.85 |
| Group III | 340 | 25.07 | 109 | 2.27 |
| Group IV | 324 | 23.24 | 104 | 2.20 |
| Group V | 345 | 25.31 | 110 | 2.25 |

| | | | | |
|---|---|---|---|---|
| ^{a} bone mineral density evaluation, mg/cm³; ^{b} bone volume fraction, %; ^{c} trabecular thickness, µm; ^{d} trabecular number, mm⁻¹. | | | | |

The results show that female subjects treated with a silicium supplement according to the invention (Group III and V) showed reduced loss of bone mineral density, better bone volume fraction, and better trabecular thickness, number and separation as compared to subjects without said silicium supplement (Group II and IV).

Subjects treated with a calcium and vitamin D3 supplement with the organic silicium solution according to the invention (Group V) showed reduced loss of bone mineral density, better bone volume fraction, and better trabecular thickness compared to subjects treated with a calcium and vitamin D3 supplement without the organic silicium solution according to the invention (Group III).

These results lead to conclude that the organic silicium solution according to the invention is effective in treatment of bone loss in female subjects subjected to ovariectomy. No intolerance to the products was observed in all treated subjects.

### EXAMPLE 5

A 100 ml orally administrable silicium solution is prepared in accordance to the instructions detailed in Example 1 and comprises purified water, silicic acid (292 mg including 86 mg of silicon), hydroalcoholic purified nettle/urtica dioïca extract (7.5 mg), flavourings (vanillin, lemon), phosphoric acid.

A topically applicable organic silicium gel is prepared in accordance to the instructions detailed in Example 1 and comprises water (90.6%), monomethylsilanetriol (0.08%), acrylamide/ammonium acrylate copolymer (2.5%), capryloyl glycine (1.8%), tromethamine (1.17%), polyisobutene (1.16%), flavouring (0.5%), polysorbate 20 (0.19%).

A group of mice was subjected to wound healing experiments. The group was devised in: one group topically treated daily with 100 mg placebo gel; one group topically treated daily with 100 mg silicium gel as described above; one group orally treated with a daily dose of 3 mL per kg body weight placebo solution; and one group orally treated with a daily dose of 3 mL per kg body weight silicium solution as described above.

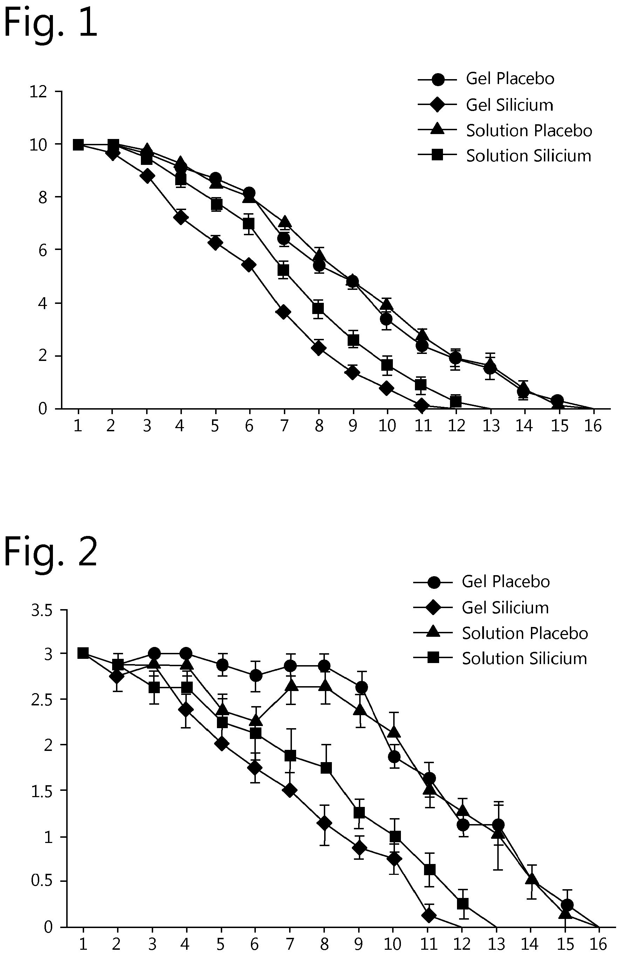

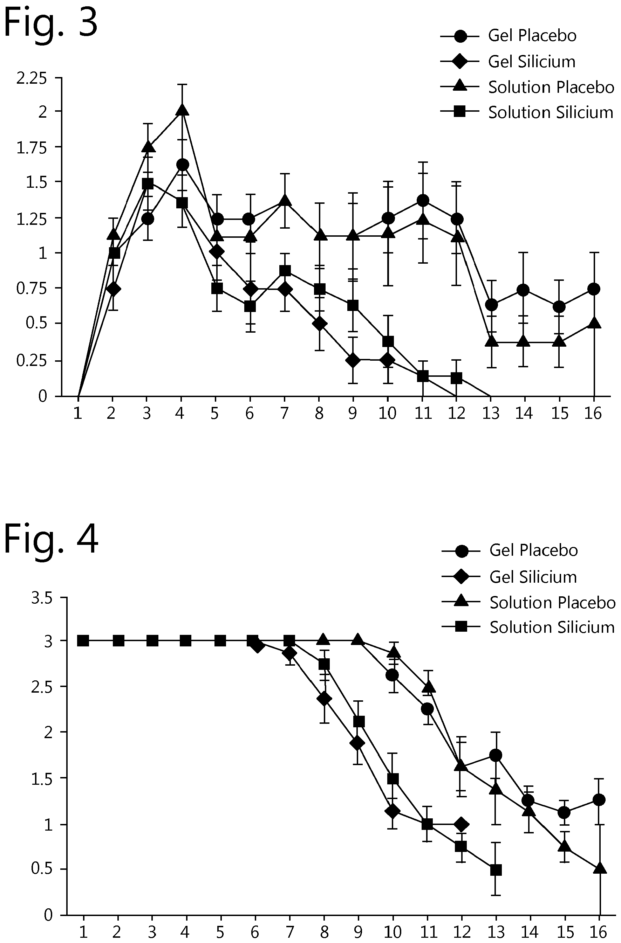

Wound healing was evaluated over a period of 16 days, and the results are depicted in Figures 1-4. The results distinctively show that wound healing is identical for placebo treated mice. In contrast, mice topically treated with an organic silicium gel or orally treated with an organic silicium solution show a distinctly faster decrease in length (Fig. 1, x-axis: treatment time in days, y-axis: wound length in mm) and width (Fig. 2, x-axis: treatment time in days, y-axis: qualitative determination of width of tissue, 0 = completely closed wound; 1 = slightly opened wound; 2 = moderately opened wound; 3 = completely opened wound) of the wound. Additionally, swelling of the tissue around the wound (Fig. 3, x-axis: treatment time in days, y-axis: qualitative determination of swelling of tissue, 0 = not swelling sore; 1 = slightly swelling sore; 2 = moderately swelling sore; 4 = completely swelling sore) disappeared faster in mice treated with organic silicium according to the invention (after about 9 days) compared to control mice (more than 13 days). The visibility of the wound (Fig. 4, x-axis: treatment time in days, y-axis: qualitative determination of visibility of wound, 0 = invisible wound; 1 = slightly visible wound; 2 = moderately visible wound; 3 = extremely visible wound) decreased faster in mice treated with organic silicium according to the invention (after about 7 days) compared to control mice (more than 10 days).

These results lead to conclude that mice topically treated daily with silicium gel or silicium solution according to the invention showed distinct faster and better wound healing compared to mice not treated with organic silicium according to the invention. No intolerance to the products was observed in all treated subjects. Thus, said silicium compositions may be effectively used for healing of wounds in a subject, i.e. for prevention of scar tissue formation and treatment of wounds resulting from a surgical procedure.

### EXAMPLE 6

The purpose of this study was to assess the effectiveness of a silicium solution according to Example 2 (15 mL three times per day) used with a topical gel according to Example 3 (twice daily application) for a period of 12 weeks in terms of improving mobility, joint comfort and ease of movement for people with joint discomfort or sensitivity in their lower limbs, compared to a different dietary supplement (15 mL three times per day) combined with a different topical gel (twice daily application).

After 12 weeks, subjects showed a decreased discomfort when walking on a flat surface, when going up or down stairs, when standing for extended periods, when getting up from or sitting down in an armchair, when crouching, when stepping over an obstacle, when doing housework or gardening and when getting out of bed. Overall, the subjects experienced a significantly reduced discomfort related to joints and bones. No intolerance to the products was observed in all treated subjects.

### EXAMPLE 7

A 100 ml orally administrable silicium solution is prepared in accordance to the instructions detailed in Example 1 and comprises purified water, silicic acid (292 mg including 86 mg of silicon), hydroalcoholic purified nettle/urtica dioïca extract (7.5 mg), flavourings (vanillin, lemon), phosphoric acid.

A topically applicable organic silicium gel is prepared in accordance to the instructions detailed in Example 1 and comprises water (90.6%), monomethylsilanetriol (0.08%), acrylamide/ammonium acrylate copolymer (2.5%), capryloyl glycine (1.8%), tromethamine (1.17%), polyisobutene (1.16%), flavouring (0.5%), polysorbate 20 (0.19%).

The purpose of this study was to assess the effectiveness of a silicium solution as described above (15 mL three times per day) used with a topical gel as described above (twice daily application) for a period of 12 weeks in terms of improving skin biomechanical properties and collagen density in a female population between 45 and 65 years old.

The results showed an improvement of the biomechanical properties of the skin characterized by a significant decrease of the firmness parameter (R0) by 10% after 28 days and by 22% after 56 days; as well as a significant increase of the biological elasticity parameter (R2) by 16% after 28 days and 14% after 56 days. Most subjects (68%) noted a more elastic skin. Furthermore, a re-densifying effect by increase of concentration of collagen in the papillary dermis by 12% after 28 days and by 15% after 56 days was observed. No intolerance to the products was observed in all treated subjects.

### EXAMPLE 8

A 100 ml orally administrable silicium solution is prepared in accordance to the instructions detailed in Example 1 and comprises purified water, silicic acid (292 mg including 86 mg of silicon), hydroalcoholic purified nettle/urtica dioïca extract (7.5 mg), flavourings (vanillin, lemon), phosphoric acid.

A topically applicable organic silicium gel is prepared in accordance to the instructions detailed in Example 1 and comprises water (90.6%), monomethylsilanetriol (0.08%), acrylamide/ammonium acrylate copolymer (2.5%), capryloyl glycine (1.8%), tromethamine (1.17%), polyisobutene (1.16%), flavouring (0.5%), polysorbate 20 (0.19%).

The purpose of this study was to assess the effectiveness of a silicium solution as described above (15 mL three times per day) used with a topical gel as described above (twice daily application) for a period of 12 weeks in terms of improving nail and hair properties in a female population between 45 and 65 years old.

The results showed an improvement of nail and hair strength in about 45% to 50% of treated subjects, a reduced loss of hairs in 77% of subjects and the absence of nail splitting. Hair and nails further were found to be less brittle in 63% and 54% of the subjects, respectively. No intolerance to the products was observed in all treated subjects.

## Claims

1. A composition comprising a stable, bioavailable silicon complex formed between silicic acid having free hydroxyl groups and at least one stabilizing agent based on phenol or polyphenol, stabilizing said free hydroxyl groups of silicic acid, for use in the prevention and/or treatment of an injury, a disease, or a disorder in a connective and/or keratinous tissue in a subject in need thereof.

2. Composition for use according to claim 1, wherein said silicic acid is orthosilicic acid having four free hydroxyl groups.

3. Composition for use according to claim 1 or 2, for use in the prevention and/or treatment of sores, wounds, ulcers, fistula or otitis.

4. Composition for use according to any of claims 1 to 3, for use in the prevention and/or treatment of collagen density loss.

5. Composition for use according to any of claims 1 to 4, for use for the prevention and/or treatment joint and bone health loss and/or improvement of joint and bone health.

6. Composition for use according to claim 4 or 5, further in combination with a supplement of calcium and/or vitamin D, and preferably in combination with alendronic acid, and a supplement of calcium and/or vitamin D.

7. Composition for use according to any of claims 1 to 6, whereby said composition is comprised in an oral dosage form.

8. Composition for use according to any of claims 1 to 7, wherein said composition is comprised in a topical or transdermal dosage form.

9. Composition for use according to claim 8, further comprising a skin permeation agent.

10. Composition for use according to any of claims 1 to 9, wherein said stabilizing agent is selected from the group comprising phenolic acids, phenols, aldehyde derivatives of phenolic acids, cinnamic aldehydes, coumarins, naphthoquinones, flavonoids, stilbenes and mixtures of one or more of the aforementioned.

11. Composition for use according to claim 10, wherein said stabilizing agent is 4-hydroxy-3-methoxybenzaldehyde.

12. Composition for use according to any of claims 1 to 11, having a pH of less than 4 and greater than or equal to 2, and a Si content of between 0.3 %o and 1.4 %o by weight per volume of the composition and a weight ratio between the content of Si and the content of stabilizing agent of between 0.1 and 1.

13. Composition for use according to claim 12, wherein said composition is a stable aqueous-alcoholic solution of said silicon complex having a pH of between 2.5 and 3.5.

14. Composition for use according to any of claims 1 to 13, further comprising one or more therapeutic agents, preferably selected from the group consisting of vitamins, antimicrobial agents, disinfectants, fungicides, anti-inflammatories, wound care products, wound healing agents, plant extracts, antibacterial agents, antifungal agents, antiviral agents, antibiotics.

15. Composition for use according to any of claims 1 to 14, wherein said composition is a formulation selected from the group consisting of liquid or semi-liquid preparations such as liniments, lotions, oil-in-water or water-in-oil emulsions such as creams, ointments or pastes, and solutions or suspensions.

## Patentansprüche

1. Eine Zusammensetzung, umfassend einen stabilen, bioverfügbaren Silicium-Komplex, der zwischen Kieselsäure mit freien Hydroxyl-Gruppen und mindestens einem Stabilisierungsmittel auf Phenol- oder Polyphenolbasis, das die freien Hydroxylgruppen von Kieselsäure stabilisiert, gebildet ist, zur Verwendung bei der Prävention und/oder Behandlung einer Verletzung, einer Erkrankung oder einer Störung in einem Binde- und/oder keratinhaltigen Gewebe eines Subjekts, das dessen bedarf.

2. Zusammensetzung zur Verwendung nach Anspruch 1, wobei die Kieselsäure Orthokieselsäure mit vier freien Hydroxyl-Gruppen ist.

3. Zusammensetzung zur Verwendung nach Anspruch 1 oder 2, zur Verwendung bei der Prävention und/oder Behandlung von wunden Stellen, Wunden, Geschwüren, Fisteln oder Ohrentzündung.

4. Zusammensetzung zur Verwendung nach einem der Ansprüche 1 bis 3, zur Verwendung bei der Prävention und/oder Behandlung von Verlust an Kollagendichte.

5. Zusammensetzung zur Verwendung nach einem der Ansprüche 1 bis 4, zur Verwendung bei der Prävention und/oder Behandlung von Verlust an Gelenk- und Knochengesundheit und/oder bei der Verbesserung der Gelenk- und Knochengesundheit.

6. Zusammensetzung zur Verwendung nach Anspruch 4 oder 5, ferner in Kombination mit einem Zusatz von Calcium und/oder Vitamin D, und vorzugsweise in Kombination mit Alendronsäure und einem Zusatz von Calcium und/oder Vitamin D.

7. Zusammensetzung zur Verwendung nach einem der Ansprüche 1 bis 6, wobei die Zusammensetzung in einer oralen Darreichungsform enthalten ist.

8. Zusammensetzung zur Verwendung nach einem der Ansprüche 1 bis 7, wobei die Zusammensetzung in einer topischen oder transdermalen Darreichungsform enthalten ist.

9. Zusammensetzung zur Verwendung nach Anspruch 8, ferner ein Hautpermeationsmittel umfassend.

10. Zusammensetzung zur Verwendung nach einem der Ansprüche 1 bis 9, wobei das Stabilisierungsmittel aus der Gruppe ausgewählt ist, die Phenolsäuren, Phenole, Aldehyd-Derivate von Phenolsäuren, Zimtsäurealdehyde, Cumarine, Naphthochinone, Flavonoide, Stilbene und Mischungen aus einem oder mehreren des Vorstehenden umfasst.

11. Zusammensetzung zur Verwendung nach Anspruch 10, wobei das Stabilisierungsmittel 4-Hydroxy-3-Methoxybenzaldehyd ist.

12. Zusammensetzung zur Verwendung nach einem der Ansprüche 1 bis 11, mit einem pH-Wert von weniger als 4 und größer als oder gleich 2 und einem Si-Gehalt zwischen 0,3 Gew.-‰ und 1,4 Gew.-‰, bezogen auf das Volumen der Zusammensetzung, und einem Gewichtsverhältnis zwischen dem Gehalt von Si und dem Gehalt von Stabilisierungsmittel zwischen 0,1 und 1.

13. Zusammensetzung zur Verwendung nach Anspruch 12, wobei die Zusammensetzung eine stabile wässrig-alkoholische Lösung des Silicium-Komplexes mit einem pH-Wert zwischen 2,5 und 3,5 ist.

14. Zusammensetzung zur Verwendung nach einem der Ansprüche 1 bis 13, ferner ein oder mehrere Therapeutika umfassend, die vorzugsweise aus der Gruppe ausgewählt sind, die aus Vitaminen, antimikrobiellen Mitteln, Desinfektionsmitteln, Fungiziden, Entzündungshemmern, Wundversorgungsprodukten, Wundheilungsmitteln, Pflanzenextrakten, antibakteriellen Mitteln, antifungalen Mitteln, antiviralen Mitteln, Antibiotika besteht.

15. Zusammensetzung zur Verwendung nach einem der Ansprüche 1 bis 14, wobei die Zusammensetzung eine Formulierung ist, die ausgewählt ist aus der Gruppe, die aus flüssigen oder halbflüssigen Zubereitungen wie Einreibemitteln, Lotionen, Öl-in-Wasser- oder Wasser-in-Öl-Emulsionen wie Cremes, Salben oder Pasten und Lösungen oder Suspensionen besteht.

## Revendications

1. Une composition comprenant un complexe de silicium biodisponible stable formé entre de l'acide silicique ayant des groupes hydroxyles libres et au moins un agent stabilisant à base de phénol ou de polyphénol, stabilisant lesdits groupes hydroxyles libres de l'acide silicique, destinée à être utilisée dans la prévention et/ou le traitement d'une blessure, d'une maladie, ou d'un trouble du tissu conjonctif et/ou kératinique chez un sujet en ayant besoin.

2. Composition pour une utilisation selon la revendication 1, dans laquelle ledit acide silicique est de l'acide orthosilicique comportant quatre groupes hydroxyles libres.

3. Composition pour une utilisation selon la revendication 1 ou 2, destinée à être utilisée dans la prévention et/ou le traitement des plaies, des blessures, des ulcères, des fistules ou des otites.

4. Composition pour une utilisation selon l'une quelconque des revendications 1 à 3, destinée à être utilisée dans la prévention et/ou le traitement de la perte de densité de collagène.

5. Composition pour une utilisation selon l'une quelconque des revendications 1 à 4, destinée à être utilisée pour la prévention et/ou le traitement de la perte de santé des articulations et des os et/ou l'amélioration de la santé des articulations et des os.

6. Composition pour une utilisation selon la revendication 4 ou 5, en outre en combinaison avec un supplément de calcium et/ou de vitamine D, et de préférence en combinaison avec de l'acide alendronique, et un supplément de calcium et/ou de vitamine D.

7. Composition pour une utilisation selon l'une quelconque des revendications 1 à 6, dans laquelle ladite composition est comprise sous une forme galénique orale.

8. Composition pour une utilisation selon l'une quelconque des revendications 1 à 7, dans laquelle ladite composition est comprise sous une forme galénique topique ou transdermique.

9. Composition pour une utilisation selon la revendication 8, comprenant en outre un agent de perméation cutanée.

10. Composition pour une utilisation selon l'une quelconque des revendications 1 à 9, dans laquelle ledit agent stabilisant est choisi dans le groupe comprenant les acides phénoliques, les phénols, les dérivés aldéhydiques des acides phénoliques, les aldéhydes cinnamiques, les coumarines, les naphtoquinones, les flavonoïdes, les stilbènes et les mélanges d'un ou plusieurs des éléments susmentionnés.

11. Composition pour une utilisation selon la revendication 10, dans laquelle ledit agent stabilisant est le 4-hydroxy-3-méthoxybenzaldéhyde.

12. Composition pour une utilisation selon l'une quelconque des revendications 1 à 11, présentant un pH inférieur à 4 et supérieur ou égal à 2, et une teneur en Si comprise entre 0,3 ‰ et 1,4 ‰ en poids par volume de la composition et un rapport pondéral entre la teneur en Si et la teneur en agent stabilisant compris entre 0,1 et 1.

13. Composition pour une utilisation selon la revendication 12, dans laquelle ladite composition est une solution aqueuse-alcoolique stable dudit complexe de silicium ayant un pH compris entre 2,5 et 3,5.

14. Composition pour une utilisation selon l'une quelconque des revendications 1 à 13, comprenant en outre un ou plusieurs agents thérapeutiques, de préférence choisis dans le groupe constitué par les vitamines, les agents antimicrobiens, les désinfectants, les fongicides, les anti-inflammatoires, les produits de soin des plaies, les agents de cicatrisation des plaies, les extraits de plantes, les agents antibactériens, les agents antifongiques, les agents antiviraux, les antibiotiques.

15. Composition pour une utilisation selon l'une quelconque des revendications 1 à 14, dans laquelle ladite composition est une formulation choisie dans le groupe constitué par les préparations liquides ou semi-liquides telles que les liniments, les lotions, les émulsions huile dans l'eau ou eau dans l'huile telles que les crèmes, les pommades ou les pâtes, et les solutions ou les suspensions.
